(19) Europäisches Patentamt
European Patent Office
Office européen des brevets

(11) EP 1 645 598 A1

(12) **EUROPEAN PATENT APPLICATION**
published in accordance with Art. 158(3) EPC

(43) Date of publication:
**12.04.2006 Bulletin 2006/15**

(21) Application number: **04747481.2**

(22) Date of filing: **14.07.2004**

(51) Int Cl.:
***C09B 43/16*** *(1968.09)* ***C09D 11/00*** *(1968.09)*
***B41J 2/01*** *(1990.01)* ***B41M 5/00*** *(1968.09)*

(86) International application number:
**PCT/JP2004/010015**

(87) International publication number:
**WO 2005/007752 (27.01.2005 Gazette 2005/04)**

(84) Designated Contracting States:
**CH DE FR GB LI**

(30) Priority: **16.07.2003 JP 2003275348**

(71) Applicant: **NIPPON KAYAKU KABUSHIKI KAISHA**
**Tokyo 102-8172 (JP)**

(72) Inventors:
- **SHIRASAKI, Yasuo,**
**Nippon Kayaku Kabushiki Kaisha**
**Kita-ku, Tokyo 115-0042 (JP)**
- **FUJII, Katsunori,**
**Nippon Kayaku Kabushiki Kaisha**
**Kita-ku, Tokyo 115-0042 (JP)**
- **NAGASAKI, Kazunobu,**
**KK Nippon Kayaku Tokyo**
**Adachi-ku,**
**Tokyo 123-0865 (JP)**

(74) Representative: **Gille Hrabal Struck Neidlein Prop Roos**
**Brucknerstrasse 20**
**40593 Düsseldorf (DE)**

(54) **DISAZO COMPOUND AND INK COMPOSITION CONTAINING THE SAME**

(57) Problems

The present invention provides an ink composition which, when applied as an ink-jet recording solution to plain paper and processed glossy paper, assumes vivid yellow color and gives printed material excellent in light fastness, gas fastness and moisture fastness.

Means for solving the problems

Ink-jet printing with an ink-jet ink composition containing a disazo compound represented by the following formula (1) can give a vivid yellow-colored product excellent in light fastness, gas fastness and moisture fastness

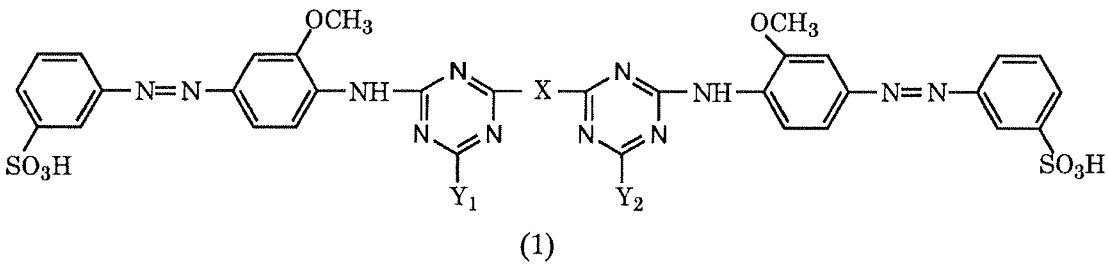

(1)

(in the formula (1), X represents specific diamine residue; and $Y_1$ and $Y_2$ each represent an alkylamino group substituted with a sulfonic acid group and/or a carboxyl group, a phenoxy group substituted with a sulfonic acid group and/or a carboxyl group and an anilino group substituted with a sulfonic acid group and/or a carboxyl group).

EP 1 645 598 A1

## Description

Technical Field

The present invention relates to a recording solution, a water-based yellow ink composition and an ink-jet recording method, and a new disazo compound.

Background of the Invention

Diverse ink jetting processes have been developed for the recording method by means of ink-jet printer, and the process respectively comprises generating ink droplets to deposit onto various recording materials (such as paper, film, cloth) for recording. The recording method by means of ink-jet printer is characterized in that it creates no machine noise due to the mechanism in which a recording head does not contact with the recording material. Such a method, being characterized by readily providing a compact, high-speed and color printer, has rapidly been spread in recent years and further propagation may be expected in the future. In order to record in color an image information or a character information displayed on a computer color monitor by means of an ink-jet printer, the information is generally printed according to subtractive color mixing of inks of four colors, namely those are yellow(Y), magenta(M), cyan(C) and black(K). In order to reproduce an printed image created by additive color mixing of R, G and B on a CRT display as faithfully as possible according to subtractive color mixing, the dyestuff to use, especially those for each Y, M and C inks, are desired to have vividness and color hues close to the standards of Y, M and C respectively. Additionally, it is required that the resulting ink composition is stable for long-term storage, and the printed image has high optical density and excellent fastness including water fastness, light fastness and gas fastness.

The use of ink-jet printers has diversely been propagated from compact ones for OA to large ones for industrial use. Therefore, more fastnesses including water fastness and light fastness are desired. The water fastness has substantially been being improved by coating inorganic micro particles such as porous silica, cationic polymer, alumina sol or special ceramics which can absorb dyestuff in ink, on a paper sheet together with PVA resin. However, the moisture fastness during storage of a printed matter such as a photograph is required to be improved further. Technology for substantially improving light fastness is not yet established. Especially among four primary colors of Y, M, C and K, a yellow dyestuff having vivid hue and excellent light fastness and moisture fastness is not available and its improvement is a critical subject.

With a digital camera becoming popular lately, there are more chances to print an image to obtain a photograph at home. Color change of such a photograph during storage caused by an oxidant gas in the air is seen as a problem. With regard to a yellow recording solution, color changing and fading caused by light (light fastness) and blot of an image caused by water and moisture (water fastness, moisture fastness) pose technical problems and many solutions have been proposed.

Patent Literatures 1 to 5, for example, disclose compounds having similar structure to that of a disazo compound of the present invention, but do not provide a yellow dyestuff which, when applied to plain paper and processed glossy paper for ink-jet recording, give vivid hue and printed material excellent in light fastness, gas fastness and moisture fastness.

Patent Literature 1: JAPANESE LAID OPEN PATENT-1992/252270
Patent Literature 2: JAPANESE LAID OPEN PATENT-1996/325493
Patent Literature 3: JAPANESE LAID OPEN PATENT-1998/279858
Patent Literature 4: JAPANESE LAID OPEN PATENT-2000/144003
Patent Literature 5: JAPANESE PUBLISHED PATENT-1980/11708

Disclosure of the Invention

Problems to be Solved by the Invention

It is an object of the present invention to provide an ink composition which, when applied to ink-jet recording and recording by a writing implement in plain paper and processed glossy paper, gives vivid hue and printed material excellent in light fastness, gas fastness and moisture fastness, and a dyestuff suitable for preparing the ink composition.

Means for Solving the Problems

The inventors of the present invention have found, after having intensively studied a way to solve the above mentioned problems, that an ink composition containing a disazo compound having specific structure can solve the

above problems, and thus completed the present invention.

In more detail, the present invention relates to:

(1) A disazo compound represented by the following formula (1), in a free acid form:

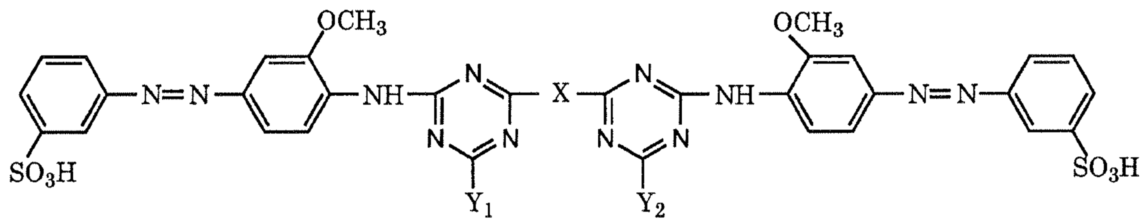

(1)

{wherein, X represents a group represented by the formula (2):

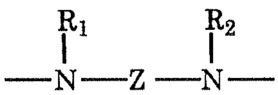

(2)

(Z in the formula (2) represents an alkylene group of 1 to 6 carbon atoms that may be substituted with one alkyl group of 1 to 3 carbon atoms depending on circumstances; $R_1$ and $R_2$ in the formula (2) represent each independently a hydrogen atom or an alkyl group of 1 to 3 carbon atoms); $Y_1$ and $Y_2$ represent each an alkylamino group substituted with a sulfonic acid group and/or a carboxyl group, a phenoxy group substituted with a sulfonic acid group and/or a carboxyl group or an anilino group substituted with a sulfonic acid group and/or a carboxyl group}.

(2) The disazo compound according to (1), wherein X in formula (1) is one selected from a group consisting of $NHC_2H_4NH$, $NHC_3H_6NH$, $NHCH\ (CH_3)CH_2NH$, $NHC_2H_4N(CH_3)$, $NHC_3H_6N(CH_3)$, $NHC\ (CH_3)_2CH_2NH$, $NHC_2H_4N\ (C_2H_5)$, $N\ (CH_3)C_2H_4N(CH_3)$ and $NHC_3H_6N(C_3H_7)$.

(3) The disazo compound according to (1) or (2), wherein X in formula (1) is $NHC_2H_4NH$.

(4) The disazo compound according to any one of (1) to (3), wherein $Y_1$ and $Y_2$ in formula (1) is $NHC_2H_4SO_3H$.

(5) A disazo compound represented by the following formula (4), in a free acid form:

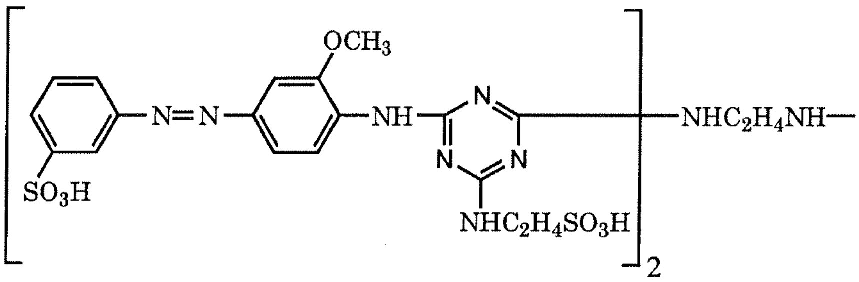

(4)

(6) A recording solution containing a aqueous medium and at least one kind of the disazo compound according to any one item of (1) to (5) .

(7) An ink composition comprising the recording solution according to (6).

(8) An ink-jet ink composition comprising the recording solution according to (6).

(9) An ink-jet recording method wherein ink droplets are discharged according to a record signal to record on recording material, characterized by using, as an ink, the ink-jet ink composition according to (8).

(10) The ink-jet recording method according to (9) wherein the recording material is an information transmitting sheet.

(11) An ink-jet printer equipped with a container containing the ink-jet ink composition according to (8) and

(12) A colored product colored by the ink-jet printer according to (11).

Effect of the Invention

The disazo compound of the present invention represented by the above formula (1) is extremely excellent in solubility in water and characterized in that an aqueous solution thereof has good keeping stability and good filtering characteristics with a membrane filter in a production process of an ink composition. The ink composition of the present invention containing the disazo compound has good storage stability without crystallization, physical property change, color change, and the like after long-term storage. A printed matter obtained by using the ink composition of the present invention as a yellow ink for ink-jet recording is excellent in light fastness, ozone fastness and moisture fastness and thus excellent ink-jet recording can be obtained. As mentioned above, the ink composition of the present invention is extremely useful as a yellow ink for ink-jet recording.

Best Modes for Carrying out the Invention

The present invention will be described in detail.
The disazo compound (a dyestuff) of the present invention is represented by the following general formula (1), in a free acid form:

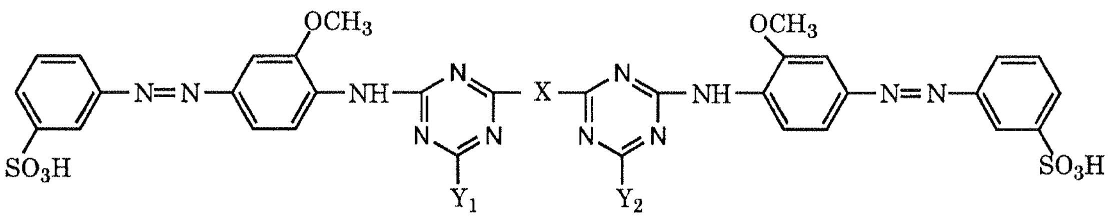

(1)

{wherein, X represents a group represented by the formula (2):

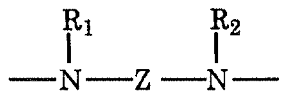

(2)

(Z in the formula (2) represents an alkylene group of 1 to 6 carbon atoms that may be substituted with one alkyl group of 1 to 3 carbon atoms depending on circumstances; $R_1$ and $R_2$ in the formula (2) represent each independently a hydrogen atom or an alkyl group of 1 to 3 carbon atoms); $Y_1$ and $Y_2$ represent each an alkylamino group substituted with a sulfonic acid group and/or a carboxyl group, a phenoxy group substituted with a sulfonic acid group and/or a carboxyl group or an anilino group substituted with a sulfonic acid group and/or a carboxyl group}.
Specific example of X in formula (1) includes, for example, $NHC_2H_4NH$, $NHC_3H_6NH$, NHCH ($CH_3$) $CH_2NH$, $NHC_2H_4N$ ($CH_3$), $NHC_3H_6N$ ($CH_3$), NHC ($CH_3$) $_2CH_2NH$, $NHC_2H_4N$ ($C_2H_5$), N ($CH_3$)$C_2H_4N$ ($CH_3$), $NHC_3H_6N$ ($C_3H_7$), $NHC_3H_6NH$, $NHC_4H_8NH$, $NHC_5H_{10}NH$, $NH(CH_2)_3C_2H_4NH$, $NH_3H_6NH_2$, $NHCH_2C_3H_6CH_2NH$, and $NHC_2H_4NH$ is especially preferable.
Specific example of $Y_1$, $Y_2$ in formula (1) includes, for example, $NHC_2H_4SO_3H$, $NHCH_2COOH$, $NHC_2H_4COOH$ and $N(CH_2COOH)_2$.

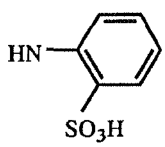

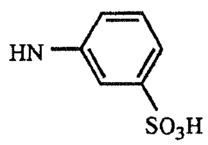

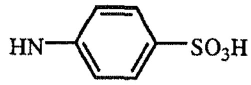

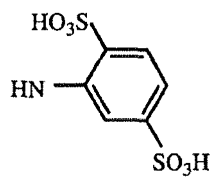

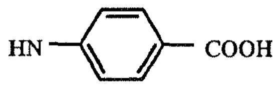

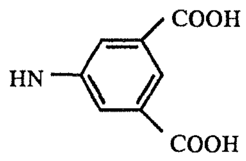

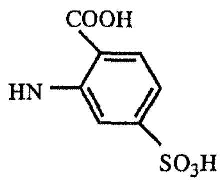

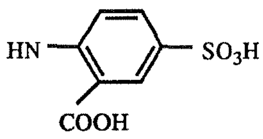

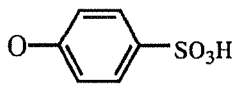

It is especially preferable when both $Y_1$ and $Y_2$ are $NHC_2H_4SO_3H$.

The compound represented by formula (1) is produced, for example, as follows. The monoazo compound represented by the following formula (A) and cyanuric chloride are condensed (primary condensation) to obtain a compound represented by the following formula (B) . The compound of the formula (B) and amines represented by the formula (C1) and/or the formula (C2) are then condensed (secondary condensation) to obtain a compound represented by the formula (D1) and/or the formula (D2). The compound represented by the formula (D1) and/or the formula (D2) and a compound represented by the formula (E) are then condensed (tertiary condensation) to obtain the object compound represented by the above formula (1). In the following formulae (C1), (C2), (D1), (D2) and (E) , $Y_1$, $Y_2$ and X represent the same as in the above formula (1) , and a sulfonic acid group is represented in a free acid form.

The above primary, secondary and tertiary condensation reactions are preferably carried out at 0 to 10°C and pH 1 to 7, 20 to 70°C and pH 3 to 9, and 80 to 95°C and pH 4 to 10, respectively. Water is usually used as a reaction medium in these reactions.

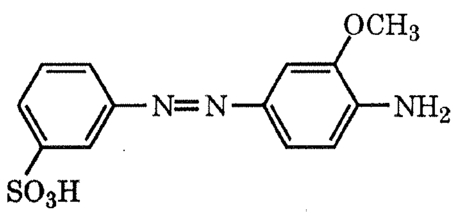

(A)

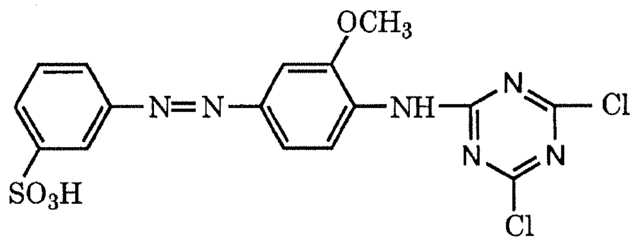

(B)

$Y_1$-H (C1)

$Y_2$-H (C2)

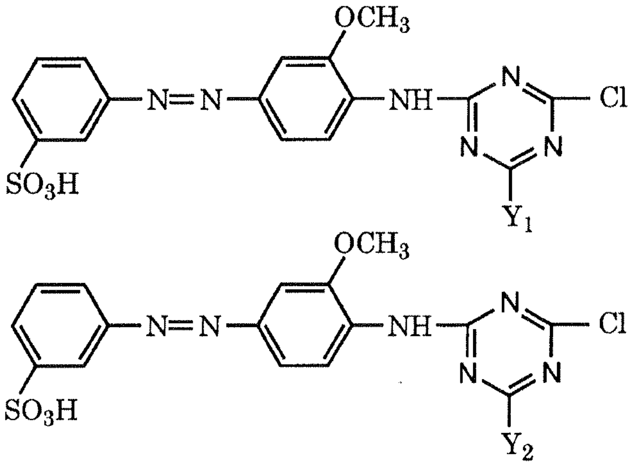

(D1)

(D2)

H-X-H (E)

Specific examples of the compounds (dyestuffs) represented by the formula (1) are shown in Table 1 below, although the compounds of the present invention are not limited thereto. A sulfonic acid group and a carboxyl group are represented in a free acid form.

Table-1

| | X | $Y_1$ | $Y_2$ |
|---|---|---|---|
| 1 | $NHC_2H_4NH$ | $NHC_2H_4SO_3H$ | $NHC_2H_4SO_3H$ |
| 2 | $NHC_2H_4NH$ | $NHCH_2COOH$ | $NHCH_2COOH$ |
| 3 | $NHC_2H_4NH$ | $NH(CH_2COOH)_2$ | $N(CH_2COOH)_2$ |
| 4 | $NHC_3H_6NH$ | $NHC_2H_4SO_3H$ | $NHC_2H_4SO_3H$ |
| 5 | $NHC_3H_6NH$ | $NHCH_2COOH$ | $N(CH_2COOH)_2$ |
| 6 | $NHCH(CH_3)CH_2NH$ | $NHC_2H_4SO_3H$ | $NHC_2H_4SO_3H$ |
| 7 | $NHC_2H_4N(CH_3)$ | $NHC_2H_4SO_3H$ | $NHC_2H_4SO_3H$ |
| 8 | $NHC_3H_6N(CH_3)$ | $NHCH_2COOH$ | $NHCH_2COOH$ |
| 9 | $NHC(CH_3)_2CH_2NH$ | $NHC_2H_4SO_3H$ | $NHC_2H_4SO_3H$ |
| 10 | $NHC_2H_4N(C_2H_5)$ | $NHC_2H_4SO_3H$ | $NHC_2H_4SO_3H$ |
| 11 | $N(CH_3)C_2H_4N(CH_3)$ | $NHCH_2COOH$ | $NHCH_2COOH$ |
| 12 | $NHC_3H_6N(C_3H_7)$ | $NHC_2H_4SO_3H$ | $NHC_2H_4SO_3H$ |
| 13 | $NHC_4H_8NH$ | $NHC_2H_4SO_3H$ | $NHC_2H_4SO_3H$ |
| 14 | $NHC_5H_{10}NH$ | $N(CH_2COOH)_2$ | $N(CH_2COOH)_2$ |
| 15 | $NH(CH_2)_3CH(CH_3)CH_2NH$ | $NHCH_2COOH$ | $NHCH_2COOH$ |
| 16 | $NHCH_2C(CH_3)_2CH_2NH$ | $NHCH_2COOH$ | $NHCH_2COOH$ |
| 17 | $NHC_6H_{12}NH$ | $NHC_2H_4SO_3H$ | $NHC_2H_4SO_3H$ |
| 18 | $NHC_3H_6N(C_3H_7)$ | $NHC_2H_4SO_3H$ | $NHCH_2COOH$ |

| No. | X | $Y_1$ | $Y_2$ |
|---|---|---|---|
| 19 | $NHC_2H_4NH$ | 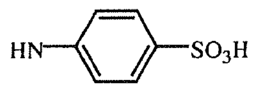 | 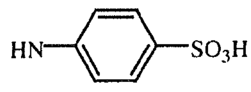 |
| 20 | $NHC_2H_4NH$ | 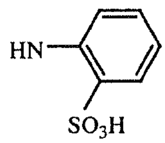 | 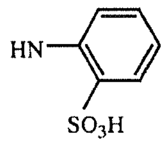 |
| 21 | $NHC_2H_4NH$ | 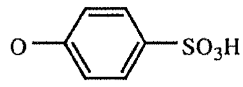 | 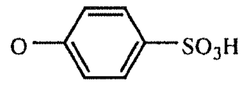 |
| 22 | $NHC_3H_6NH$ | 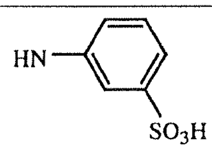 | 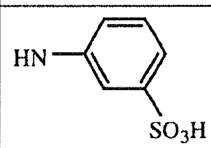 |
| 23 | $NHC_3H_6NH$ | 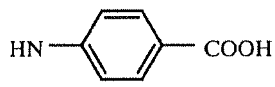 | 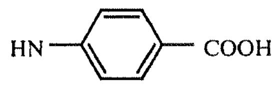 |
| 24 | $NHCH(CH_3)CH_2NH$ | 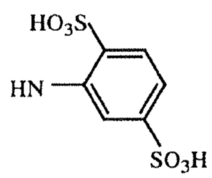 | 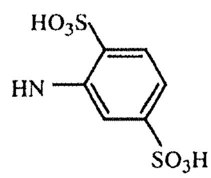 |
| 25 | $NHC_2H_4N(CH_3)$ | 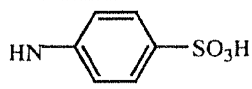 | 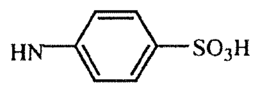 |
| 26 | $NHC_3H_6N(CH_3)$ | 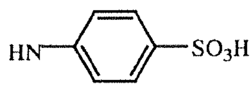 | 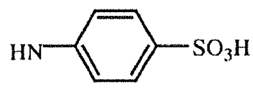 |
| 27 | $NHC(CH_3)_2CH_2NH$ | 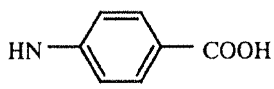 | 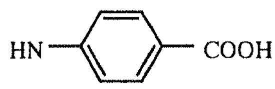 |
| 28 | $NHC_2H_4N(C_2H_5)$ | 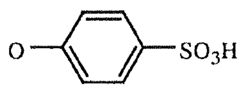 | 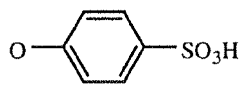 |
| 29 | $N(CH_3)C_2H_4N(CH_3)$ | 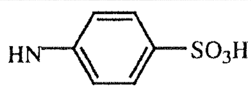 | 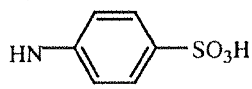 |
| 30 | $NHC_3H_6N(C_3H_7)$ | 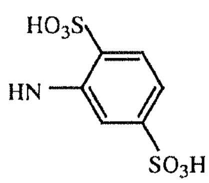 | 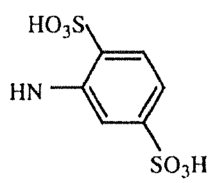 |

The above compound of the present invention can be used in a free acid form or a salt form thereof. The salt includes an alkali metal salt or an alkylamine salt, an alkanolamine salt, or an ammonium salt represented by the following formula (3):

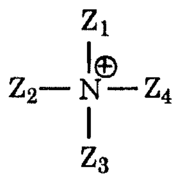

(3)

(wherein, $Z_1$, $Z_2$, $Z_3$ and $Z_4$ represent each independently a hydrogen atom, an alkyl group, a hydroxyalkyl group or a hydroxyalkoxyalkyl group).

Preferable salts include an alkali metal salt such as a sodium salt, a potassium salt and a lithium salt, an alkanolamine salt such as a monoethanolamine salt, a diethanolamine salt, a triethanolamine salt, a monoisopropanolamine salt, a diisopropanolamine salt and a triisopropanolamine salt and an ammonium salt. A sodium salt, for example, is obtained as a wet cake by adding sodium chloride into the reaction solution of the compound obtained above to salt out and then filtering. The above wet cake is dissolved again in water and then hydrochloric acid is added thereto to adjust pH 1 to 2 and thus obtained crystals are filtered to obtain a free acid (or a partly remaining sodium salt). The wet cake in the free acid form is alkalified by the addition of, for example, potassium hydroxide, lithium hydroxide and ammonia water under stirring along with water to obtain a potassium salt, a lithium salt and an ammonium salt, respectively.

The ink composition of the present invention is prepared by dissolving the compound of the present invention represented by the above formula (1) or a salt thereof in water or an aqueous solvent (water containing a water-soluble organic solvent (including a solution assistant, same hereinafter) to be described later). The compound represented by the above formula (4) is especially preferably used. The approximate pH of the ink composition is preferably 6 to 11. When the ink composition is used for an ink-jet printer, a dyestuff component having low content of an inorganic compound such as a chloride and a sulfate of a metal cation is preferable and the approximate standard of the content is, for example, 1% by mass or less of the total amount of sodium chloride and sodium sulfate. For preparing a compound (a dyestuff component) having low content of inorganic compounds, a desalting treatment by, for example, an ordinary method using a reverse osmosis membrane or a method where a dried material or a wet cake of the dyestuff component of the present invention is stirred in a mixed solvent of methanol and water, filtered and dried in required times is repeated. The ink composition is used as a yellow ink, but may be mixed with other dyestuffs within a range not to impair the spirit of the present invention.

The ink composition of the present invention is prepared using water as a medium. The compound of the above formula (1) obtained by the method described above is contained usually by 0.3 to 8% by mass in the ink composition of the present invention. The water-soluble organic solvent is also contained as needed in the ink composition of the present invention within a range not to impair the effect of the present invention. The water-soluble organic solvent may be used together with a dye-dissolving agent, a dryness inhibitor (a wetting agent), a viscosity modifier, a penetrating agent, a surface tension modifier, an antifoaming agent, and the like. Other ink preparation agents include, for example, known additives such as a preservative and fungicide, a pH adjusting agent, a chelating agent, an antirust agent, an ultraviolet absorber, a viscosity modifier, a dye-dissolving agent, a fading inhibitor, an emulsion stabilizer, a surface tension modifier, an antifoaming agent, a dispersing agent and a dispersion stabilizer. Content of the water-soluble organic solvent is usually 0 to 60% by mass and preferably 10 to 50% by mass, while content of the ink preparation agent is usually 0 to 20% by mass and preferably 0 to 15% by mass.

Example of aqueous organic solvent includes, for example, alkanol having carbon number C1 to C4 such as methanol, ethanol, n-propanol, isopropanol, n-butanol, isobutanol, sec-butanol, tert-butanol; carboxylic acid amide such as N,N-dimethylformamide, or N,N-dimethylacetamide; heterocyclic ketone such as 2- pyrrolidone, N-methyl-2- pyrrolidone, 1,3-dimethylimidazolidine-2-one, or 1,3-dimethylhexahydropyrimido-2-one; ketone or ketoalcohol such as acetone, methyl ethyl ketone, 2-methyl-2-hydroxypentane-4-one; cyclic ether such as tetrahydrofuran, dioxane; monomer, oligomer or polyalkylene glycol or thioglycol having carbon number C2 to C6 alkylene unit such as ethylene glycol, 1,2- or 1,3-propylene glycol, 1,2- or 1,4-butylene glycol, 1,6-hexylene glycol, diethylene glycol, triethylene glycol, tetraethylene glycol, dipropylene glycol, thiodiglycol, polyethylene glycol, polypropylene glycol; or polyol(triol) such as glycerin, hexane-1,2,6-triol; (C1 to C4)alkyl ether of polyhydric alcohol such as ethylene glycol monomethyl ether, or ethylene glycol monoethyl ether, diethylene glycol monomethyl ether, or diethylene glycol monoethyl ether, or triethylene glycol monomethyl ether, or triethylene glycol monoethyl ether; $\gamma$-butyrolactone, or dimethylsulfoxide.

Among them, isopropanol, glycerin, mono, di or triethylene glycol, dipropylene glycol, 2-pyrrolidone, N-methyl-2-pyrrolidone is preferable. Isopropanol, glycerin, diethylene glycol, 2-pyrrolidone are more preferable. The aqueous organic solvent can be used alone or in combination with other solvents.

Preservative and fungicide includes, for example, organosulfur type, organonitrogensulfur type , organohalogen

type, haloarylsulfone type, iodopropargyl type, N-haloalkylthio type, benzthiazole type, nitrile type, pyridine type, 8-oxyquinoline type, benzothiazole type, isothiazoline type, dithiol type, pyridine oxide type, nitropropane type, organic tin type, phenol type, quaternary ammonium type, triazine type, thiadiazine type, anilide type, adamantane type, dithiocarbamate type, brominated indanone type, benzyl bromoacetate type, inorganic salt compound.
Organohalogen type compound includes, for example, pentachlorophenol sodium; pyridine oxide type compound includes, for example, 2-pyridinethiol-1-oxide sodium; inorganic salt compound includes anhydrous sodium acetate; isothiazoline type compound includes, for example, 1,2-benzisothiazoline-3-one, 2-n-octyl-4-isothiazoline-3-one, 5-chloro-2-methyl-4-isothiazoline-3-one, 5-chloro-2-methyl-4-isothiazoline-3-one magnesium chloride, 5-chloro-2-methyl-4-isothiazoline-3-one calcium chloride, 2-methyl-4-isothiazoline-3-one calcium chloride. The other preservative and fungicide includes, for example, sodium sorbate, sodium benzoate (Proxel GXL (S), Proxel XL-2 (S), etc. manufactured by Abesia Co., Ltd.).

Any substance would be optionally used as pH adjusting agent if this substance makes it possible to control pH of ink composition in the range of 6. 0 to 11. 0 to improve the preservation stability of ink composition.

pH adjusting agent includes, for example, alkanol amine such as diethanol amine, triethanol amine; alkali metal hydroxide such as lithium hydroxide, sodium hydroxide, potassium hydroxide; ammonium hydroxide, or alkali metal carbonate such as lithium carbonate, sodium carbonate, potassium carbonate.

Chelating reagent includes, for example, tetrasodium ethylenediamine tetraacetate, trisodium nitrilotriacetate, trisodium hydroxyethylethylenediamine triacetate, pentasodium diethylenetriamine pentaacetate, disodium uramildiacetate. Rust preventive agent includes, for example, hydrogensulfite, sodium thiosulfate, ammonium thioglycollate, diisopropylammonium nitrite, pentaerythritol tetranitrate, dicyclohexylammonium nitrite.

Ultraviolet absorber includes, for example, benzophenone type compounds, benzotriazole type compounds, cinnamic acid type compounds, stilbene type compounds, or benzoxazole type compounds which emit a fluorescence by absorbing ultraviolet ray, what is called fluorescent brightners.

Viscosity modifier includes aqueous organic solvent, and aqueous high molecular compound such as polyvinyl alcohol, cellulose derivative, polyamine, polyimine. Dye solubilizing agent includes, for example, urea, $\varepsilon$-caprolactam, ethylene carbonate.

Fading inhibitor can be used to improve the storage stability of image. Various organic or metal complex fading-inhibitors can be used as a fading-inhibitor. Organic fading-inhibitor includes hydroquinones, alkoxyphenols, dialkoxyphenols, phenols, anilines, amines, indanes, chromans, alkoxyanilines, heterocycles. Metal complex includes nickel complex, zinc complex.

Surface tension modifier includes a surface active agent, for example, anionic surface active agent, ampholytic surface active agent, cationic surface active agent, nonionic surface active agent and so on. Anionic surface active agent includes alkylsulfo carboxylate, $\alpha$-olefin sulfonate, polyoxyethylene alkyl ether acetate, N-acylamino acid and its salt, N-acylmethyl taurine, alkyl sulfate polyoxyalkyl ether sulfate, alkyl sulfate polyoxyethylene alkyl ether phosphate, rosin acid soap, castor oil sulfate, lauryl alcohol sulfate, alkylphenol type phosphate, alkyl type phosphate, alkyl aryl sulfonate, diethyl sulfosuccinate, diethylhexyl sulfosuccinate, dioctyl sulfosuccinate. Cationic surface active agent includes 2-vinylpyridine derivatives, poly (4-vinylpyridine) derivatives. Ampholytic surface active agent includes lauryldimethylaminoacetic acid betaine, 2-alkyl-N-carboxymethyl-N-hydroxyethyl imidazolinium betaine, palm oil fatty acid amide propyldimethylamino acetic acid betaine, polyoctylpolyaminoethyl glycine, and other imidazoline derivatives.

Nonionic surface active agent includes ether type compounds such as polyoxyethylene nonylphenyl ether, polyoxyethylene octylphenyl ether, polyoxyethylene dodecylphenyl ether, polyoxyethylene octylphenyl ether, polyoxyethylene oleyl ether, polyoxyethylene lauryl ether, polyoxyethylene alkyl ether, polyoxyaralkyl alkyl ether; ester type compounds such as polyoxyethylene oleic acid, polyoxyethylene oleate, polyoxyethylene distearate, sorbitan laurate, sorbitan monosterate, sorbitan monooleate, sorbitan sesquioleate, polyoxyethylene monooleate, polyoxyethylene stearate; acetylene glycol type compounds such as 2,4,7,9-tetramethyl-5-decyn-4,7-diol, 3,6-dimethyl-4-octyn-3,6-diol, 3,5-dimethyl-1-hexyn-3-ol (for example, Surfynol-104E, 104PG50, 82,465; Olfin STG, etc. manufactured by Nissin Chemical Industry Co.,Ltd.). The ink preparation agent can be used alone or in combination with other modifiers. Surface tension of ink composition in this invention is usually 25 to 70 mN/m, and is preferably 25 to 60 mN/m. Viscosity of ink composition in this invention is regulated 30 mPa·s or less, and preferably 20 mPa·s or less.

The ink composition of the present invention is prepared by adding the compound of the above formula (1), and as necessary, the above water-soluble organic solvent and the ink preparation agent to impurity-free water such as distilled water and mixing these and mixing. Impurities may be filtered off from the ink composition obtained thus, if necessary.

Recording material to be used in the ink-j et recording method of the present invention includes, for example, an information transmitting sheet such as paper and film, fiber and leather. The information transmitting sheet is preferably surface-treated, specifically provided with an ink receiving layer. The ink receiving layer is provided by, for example, impregnating a cationic polymer into the substrate mentioned above or coating it on the above substrates with a cationic polymer, or coating inorganic particles such as porous silica, alumina sol and special ceramics that are able to adsorb

dyestuffs in ink, together with a hydrophilic polymer such as polyvinyl alcohol and polyvinyl pyrrolidone on the substrate mentioned above. Such sheets provided with these ink receiving layers are usually called a professional paper (film) for ink-jet use or glossy paper (film) and are available on the market as, for example, Pictorico (made by Asahi Glass Company), Color BJ paper, Color BJ photofilm sheet (both made by Canon Inc.), Paper for color image jet (made by Sharp Corporation), Glossy film professional for superfine use (made by Seiko Epson Corporation) and Pictafine (made by Hitachi Maxell Corporation) . Plain paper without such an ink receiving layer can naturally be used.

With regard to fiber, cellulose fiber or polyamide fiber such as nylon, silk and wool is preferable, and nonwoven fabric and cloth are preferable. A dyestuff can be fixed inside fiber by applying the ink composition of the present invention to the fiber, preferably using an ink-jet method and then adding a fixing step for wet-heating (for example, at about 80 to 120°C) or dry-heating (for example, at about 150 to 180°C). The dyed fiber obtained thus has excellent vividness, light fastness and washing fastness.

The ink-jet printer of the present invention is equipped with a container containing the above water-based ink composition at the ink tank section thereof. The colored product of the present invention is colored with the above ink composition containing the disazo compound represented by the above formula (1) or the salt thereof.

The ink composition containing the disazo compound of the present invention can give recorded material especially excellent in light fastness and also excellent in ozone fastness and moisture fastness. It gives a greenish yellow color, which has suitable hue as a yellow color for ink-jet recording. Use of the above ink composition along with other inks such as magenta and cyan can give colors of a broad visible range. Use of the above ink composition along with known magenta, cyan and black excellent in light fastness, ozone fastness and moisture fastness can give recorded material excellent in light fastness, ozone fastness and moisture fastness.

Examples

The present invention will be described more specifically with Examples. "Parts" and "%" in the description are based on mass unless otherwise stated.

Example 1

Cyanuric chloride of 18.4 parts and 4-(3-sulfophenylazo)-2-methoxyaniline of 30.1 parts were subjected to primary condensation at pH 5 to 6.5 and at 3 to 10°C in an aqueous solvent. Then, taurine of 13.5 parts was added to the reaction mixtures and secondary condensation carried out at pH 6 to 9 and at 40 to 70°C. After heated to 80°C, the product of the secondary condensation was subjected to tertiary condensation at pH 8 to 10 and at 80 to 90°C by adding ethylenediamine of 4 parts dropwise in 15 to 30 minutes. As a pH adjusting agent, a 10% aqueous solution of sodium carbonate was used in the primary, secondary and tertiary condensation. Thus obtained product was subjected to salting-out preferably at not higher than 60°C and then controlled to pH 2 to 4 with hydrochloric acid, followed by filtration to obtain 54 parts of a disazo compound ($\lambda_{max}$ = 391 nm in water) in a free acid form represented by the following formula (4).

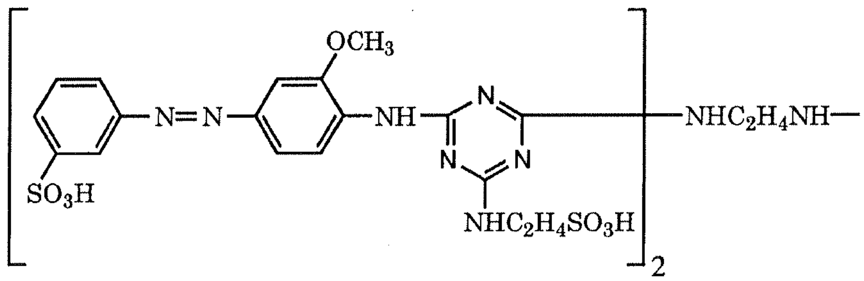

(4)

Example 2

Cyanuric chloride of 18.4 parts and 4-(3-sulfophenylazo)-2-methoxyaniline of 30.1 parts were subjected to primary condensation at pH 5 to 6.5 and at 3 to 10°C in an aqueous solvent. Then, 5-sulfoanthranilic acid of 23.9 parts was added to the reaction mixtures and secondary condensation carried out at pH 6 to 9 and at 40 to 70°C. After heated to 80°C, the product of the secondary condensation was subjected to tertiary condensation at pH 8 to 10 and at 80 to 90°C by adding ethylenediamine of 4 parts dropwise in 15 to 30 minutes. As a pH adjusting agent, a 10% aqueous solution of sodium carbonate was used in the primary, secondary and tertiary condensation. Thus obtained product was

subjected to salting-out preferably at not higher than 60°C and then controlled to pH 2 to 4 with hydrochloric acid, followed by filtration to obtain 64 parts of a disazo compound ($\lambda_{max}$ = 392 nm in water) in a free acid form represented by the following formula (5).

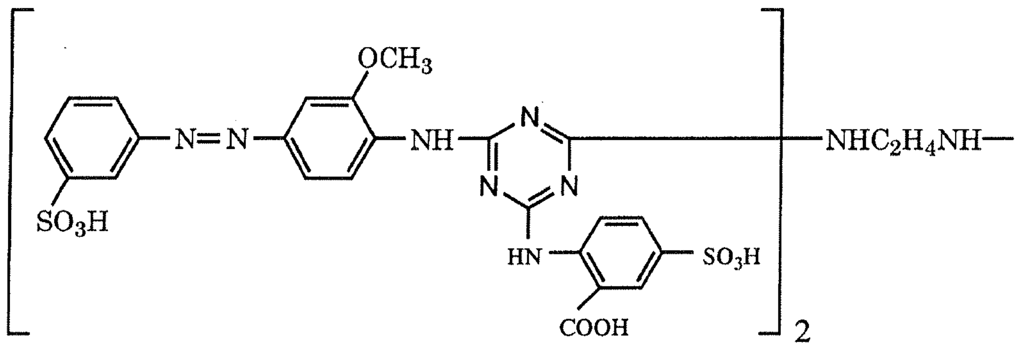

(5)

Example 3

(A) Preparation of ink

The solution of the composition shown in Table 2 below, containing the disazo compound (a dyestuff component) obtained in Example 1 or 2 was prepared and filtered with a 0.45 μm membrane filter to obtain each water-based yellow ink composition for ink-jet printing. Ion-exchange water was used. Water and ammonium hydroxide were added so that the pH of the ink composition is 8 to 10 and the total amount is 100 parts.

Table 2

| | |
|---|---|
| Each dyestuff component obtained in Example 1 or 2 (subjected to desalting) | 3.0 parts |
| Water + ammonium hydroxide | 77.9 parts |
| Glycerin | 5.0 parts |
| Urea | 5.0 parts |
| N-methyl-2-pyrrolidone | 4.0 parts |
| IPA (isopropyl alcohol) | 3.0 parts |
| Butylcarbitol | 2.0 parts |
| Surface active agent (SURFYNOL 104PG 50 made by Nissin Chemical Industry Co. Ltd.) | 0.1 parts |
| Total | 100.0 parts |

(B) Ink-jet printing

Ink-jet recording is carried out on four kinds of recording materials of plain paper, Professional photo paper (PR-101 (made by Canon Inc.)), Photo glossy film (HG-201 (made by Canon Inc.)) and Paper for PM photo <gloss> (made by SEIKO EPSON CORP.), using an ink-jet printer (Trade name: BJ S-630 from Canon Inc.) (hereinafter, described as follows, PR: Professional photo paper, HG: Photo glossy film, PM: Paper for PM photo).

(C) Evaluation of recorded image

(1) Evaluation of hue

Hue and vividness of a recorded image: L*, a*, and b* values of the recorded papers were measured using a colorimetric system (Gretag Macbeth SpectroEye: made by GRETAG Corp. ) . Vividness was calculated from chromaticity (a*, b*) using the equation: $C^* = ((a^*)^2 + (b^*)^2)^{1/2}$ and evaluated.
The results are shown in Table 3.

(2) Test for light fastness

The recorded image was irradiated at 24°C and 60%RH for 50 hours using a xenon weather meter (made by Atlas Corp. ) . Densities (D values) of the images before and after the irradiation were measured by means of the above colorimetric system.

Residual ratio (%) = D value after irradiation / D value before irradiation

The calculated results are shown in Table 3.

(3) Test for ozone fastness

A sample piece having a recorded image printed on the sample piece was left for standing at 24°C, 12 ppm and 60%RH for 2 hours using an ozone weather meter (Model OMS-H made by Suga Test Instruments Co., Ltd.). Densities (D values) before and after the test were measured.

Residual ratio (%) = D value after irradiation/ D value before irradiation

The calculated results are shown in Table 3.

(4) A sample piece having a recorded image printed on the sample piece for a moisture fastness test was left for standing at 50°C and 90%RH for 150 hours in a thermo-hygrostat (made by Ouyou giken sangyou Corp.). Bleeding degrees before and after the test was evaluated by visual inspection. The results of evaluation were classified as follows and are shown in Table 3.

○ : No bleeding is observed.
Δ : Bleeding is a little observed.
× : Bleeding is seriously observed.

Test results on hue, light fastness, ozone fastness and moisture fastness of a recorded image are shown in Table 3. The results of evaluation of the ink composition prepared using the compound obtained in Example 1 are referred to Evaluation Example 1, and similarly the results of evaluation of the ink composition prepared using the compound obtained in Example 2 are referred to Evaluation Example 2. The compound represented by the following formula (6) and the compound represented by the following formula (7) were used as Comparative Example 1 and Comparative Example 2 and those were evaluated at the same optical density as those of Evaluation Examples 1 and 2 in the composition shown in Table 2. The results were shown in Table 3. Data show evaluation results under the condition that density (D value) is about 1.0 in gradation printing.

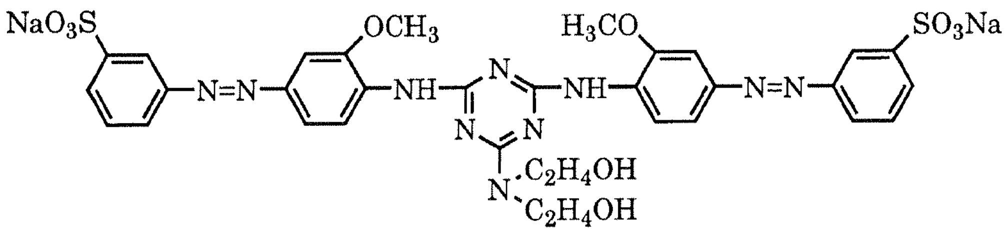
(6)

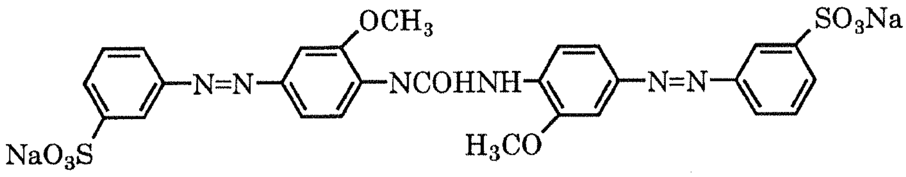

(7)

Table 3

| | Recording Material | Vividness C* | Hue | | | Light Fastness | Ozone Fastness | Moisture Fastness |
|---|---|---|---|---|---|---|---|---|
| | | | L* | a* | b* | | | |
| Evaluation Example 1 | Plain Paper | 70.9 | 89.6 | -3.1 | 70.8 | 97 | 99 | ○ |
| | PR | 58.4 | 94.8 | -8.4 | 57.8 | 98 | 93 | ○ |
| | HG | 55.0 | 93.9 | -8.5 | 54.3 | 91 | 91 | ○-Δ |
| | PM | 54.1 | 95.1 | -10.2 | 53.1 | 95 | 96 | ○ |
| Evaluation Example 2 | Plain Paper | 71.6 | 89.0 | -1.6 | 71.6 | 97 | 99 | ○ |
| | PR | 57.9 | 95.0 | -8.6 | 57.3 | 96 | 94 | ○ |
| | HG | 56.4 | 94.1 | -7.8 | 55.9 | 88 | 92 | ○ |
| | PM | 58.4 | 95.1 | -10.4 | 57.5 | 92 | 96 | ○ |
| Comparative Example 1 | Plain Paper | 62.7 | 89.1 | -1.4 | 62.7 | 78 | 99 | ○ |
| | PR | 60.9 | 95.2 | -8.4 | 60.3 | 80 | 89 | ○ |
| | HG | 61.9 | 94.4 | -8.1 | 61.4 | 67 | 91 | ○-Δ |
| | PM | 62.2 | 94.6 | -9.8 | 61.4 | 86 | 97 | ○-Δ |
| Comparative Example 2 | Plain Paper | 68.6 | 88.7 | -0.4 | 68.6 | 81 | 98 | ○ |
| | PR | 64.9 | 95.4 | -8.4 | 64.4 | 82 | 82 | ○ |
| | HG | 64.1 | 94.0 | -7.7 | 63.6 | 63 | 74 | Δ |
| | PM | 68.9 | 95.0 | -9.8 | 68.2 | 94 | 97 | × |

Light fastness and ozone fastness are shown in residual ratio %.

As apparent from Table 3, it can be understood that judging from a* values and the b* values, the Evaluation Examples show similar hue to the Comparative Examples, and judging from C* values, the Evaluation Examples show similar vividness to the Comparative Examples and in plain paper, the Evaluation Examples are more vivid than the Comparative Examples. It can be said that the Evaluation Examples 1 and 2 show vivid yellow similar to the dyestuffs of the Comparative Examples 1 and 2, which are typical yellow dyestuffs used for ink-jet printing. With regard to light fastness, the Evaluation Examples 1 and 2 have higher residual ratios, showing remarkable improvement. With regard to ozone fastness and moisture fastness, too, the Evaluation Examples 1 and 2 are better than the Comparative Examples 1 and 2. It can be understood that the disazo compound of the present invention has preferable hue as a yellow dyestuff for ink-jet printing and is a compound suitable for further improving storage stability of printed matter.

The disazo compound of the present invention is superior in ink properties as a whole and has stable high-quality in each medium (a recording agent). In addition, each dyestuff obtained in Examples 1 and 2 has solubility of not less than 100 g/l in water under an alkaline condition (pH = 8 - 9), and thus can give a stable ink as a dyestuff for ink-jet printing and also can give a high-concentration ink. Therefore, the dyestuff has a broad use and is a compound easy-to-use.

Industrial Applicability

The ink composition of the present invention can be prepared safely and inexpensively as various recording solutions, especially a yellow-ink recording solution for an ink-jet printer, and an aqueous solution thereof is excellent in storage stability and printing properties. The printed matter shows vivid yellow color and is superior in light fastness, ozone fastness and moisture fastness.

## Claims

1. A disazo compound represented by the following formula (1), in a free acid form:

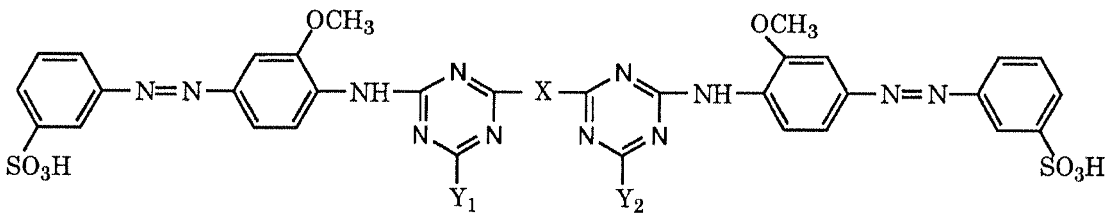

(1)

{wherein, X represents a group represented by the formula (2):

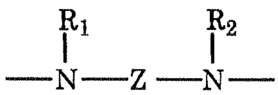

(2)

(Z in the formula (2) represents an alkylene group of 1 to 6 carbon atoms that may be substituted with one alkyl group of 1 to 3 carbon atoms depending on circumstances; $R_1$ and $R_2$ in the formula (2) represent each independently a hydrogen atom or an alkyl group of 1 to 3 carbon atoms); $Y_1$ and $Y_2$ represent each an alkylamino group substituted with a sulfonic acid group and/or a carboxyl group, a phenoxy group substituted with a sulfonic acid group and/or a carboxyl group or an anilino group substituted with a sulfonic acid group and/or a carboxyl group}.

2. The disazo compound according to claim 1, wherein X represented in formula (1) is one selected from a group consisting of $NHC_2H_4NH$, $NHC_3H_6NH$, $NHCH(CH_3)CH_2NH$, $NHC_2H_4N(CH_3)$, $NHC_3H_6N(CH_3)$, $NHC(CH_3)_2CH_2NH$, $NHC_2H_4N(C_2H_5)$, $N(CH_3)C_2H_4N(CH_3)$, and $NHC_3H_6N(C_3H_7)$.

3. The disazo compound according to Claim1 or Claim2, wherein X represented in formula (1) is $NHC_2H_4NH$.

4. The disazo compound according to any one of Claim1 to Claim3, wherein $Y_1$ and $Y_2$ represented in formula (1) is $NHC_2H_4SO_3H$.

5. A disazo compound represented by the following formula (4), in a free acid form:

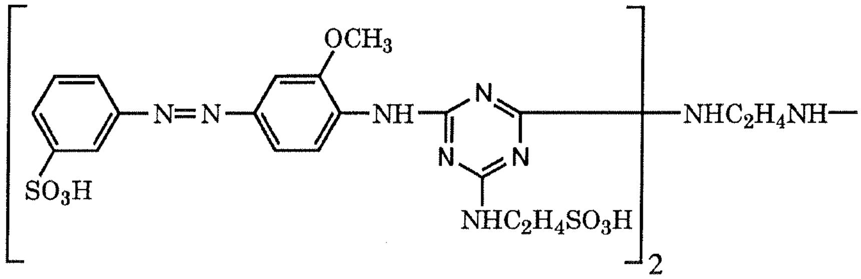

(4)

6. A recording solution containing an aqueous medium and at least one kind of the disazo compound according to any one Claims 1 to 5.

7. An ink composition comprising the recording solution according to Claim 6.

8. An ink composition for ink-jet recording, comprising the recording solution according to Claim 6.

9. An ink-jet recording method wherein ink droplets are discharged according to a record signal to record on recording material, **characterized by** using, as an ink, the ink composition for ink-jet recording according to Claim 8.

10. The ink-jet recording method according to Claim 9 wherein the recording material is an information transmitting sheet.

11. An ink-jet printer equipped with a container containing the ink composition for ink-jet recording according to Claim 8.

12. A colored product colored by the ink-jet printer according to Claim 11.

**INTERNATIONAL SEARCH REPORT**

International application No.
PCT/JP2004/010015

A. CLASSIFICATION OF SUBJECT MATTER
Int.Cl[7] C09B43/16, C09D11/00, B41J2/01, B41M5/00

According to International Patent Classification (IPC) or to both national classification and IPC

B. FIELDS SEARCHED

Minimum documentation searched (classification system followed by classification symbols)
Int.Cl[7] C09B43/16, 62/08-62/095, C09D11/00-11/20, B41J2/01, B41M5/00

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 2000-144003 A (Mitsubishi Chemical Corp.), 26 May, 2000 (26.05.00), Claims; Par. Nos. [0001], [0035]I-(2) | 1-12 |
| Y | JP 2001-19881 A (Nippon Kayaku Co., Ltd.), 23 January, 2001 (23.01.01), Claims; Par. Nos. [0024] to [0025] | 1-12 |
| Y | JP 4-233975 A (Imperial Chemical Industries PLC.), 21 August, 1992 (21.08.92), Claims; Par. Nos. [0001], [0018], [0021] | 1-12 |

[X] Further documents are listed in the continuation of Box C. [X] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
21 September, 2004 (21.09.04)

Date of mailing of the international search report
12 October, 2004 (12.10.04)

Name and mailing address of the ISA/
Japanese Patent Office

Facsimile No.

Authorized officer

Telephone No.

Form PCT/ISA/210 (second sheet) (January 2004)

INTERNATIONAL SEARCH REPORT

International application No. PCT/JP2004/010015

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-192930 A (Nippon Kayaku Co., Ltd.), 09 July, 2003 (09.07.03), Claims | 1-12 |
| A | JP 2002-285022 A (Nippon Kayaku Co., Ltd.), 03 October, 2002 (03.10.02), Claims | 1-12 |
| A | JP 10-279858 A (Mitsubishi Chemical Corp.), 20 October, 1998 (20.10.98), Claims | 1-12 |
| A | JP 8-325493 A (Ricoh Co., Ltd.), 10 December, 1996 (10.12.96), Claims | 1-12 |

Form PCT/ISA/210 (continuation of second sheet) (January 2004)

**INTERNATIONAL SEARCH REPORT**
Information on patent family members

International application No.
PCT/JP2004/010015

| | | |
|---|---|---|
| JP 2000-144003 A | 2000.05.26 | (Family: none) |
| JP 2001-19881 A | 2001.01.23 | (Family: none) |
| JP 4-233975 A | 1992.08.21 | EP 468647 A<br>AU 9180175 A<br>US 5268459 A<br>DE 69117828 E<br>ES 2084111 T3<br>KR 153007 B1 |
| JP 2003-192930 A | 2003.07.09 | WO 03/027185 A1 |
| JP 2002-285022 A | 2002.10.03 | WO 2004/026964 A1 |
| JP 10-279858 A | 1998.10.20 | (Family: none) |
| JP 8-325493 A | 1996.12.10 | US 5622550 A |

Form PCT/ISA/210 (patent family annex) (January 2004)